(19) Europäisches Patentamt
European Patent Office
Office européen des brevets

(11) **EP 3 540 435 B1**

(12) **EUROPÄISCHE PATENTSCHRIFT**

(45) Veröffentlichungstag und Bekanntmachung des Hinweises auf die Patenterteilung:
**01.07.2020 Patentblatt 2020/27**

(51) Int Cl.:
**G01N 33/24** *(2006.01)*    **B01J 21/12** *(2006.01)*
**G01N 25/22** *(2006.01)*

(21) Anmeldenummer: **18161769.7**

(22) Anmeldetag: **14.03.2018**

(54) **VERFAHREN ZUR BESTIMMUNG DER KONZENTRATION AN SAUREN ZENTREN VON FESTSTOFFEN**

METHOD FOR DETERMINING THE CONCENTRATION OF ACIDIC SITES OF SOLIDS

PROCÉDÉ DE DÉTERMINATION DE CONCENTRATION DES CENTRES ACIDES DANS LES MATIÈRES SOLIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**18.09.2019 Patentblatt 2019/38**

(73) Patentinhaber: **Evonik Operations GmbH**
**45128 Essen (DE)**

(72) Erfinder:
• **NADOLNY Fabian**
**59821 Arnsberg (DE)**
• **PEITZ, Stephan**
**45739 Oer-Erkenschwick (DE)**
• **STOCHNIOL, Guido**
**45721 Haltern am See (DE)**
• **FRANKE, Robert**
**45772 Marl (DE)**
• **QUANDT, Thomas**
**45772 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
• O. KRESNAWAHJUESA ET AL: "A Simple, Inexpensive, and Reliable Method for Measuring Brønsted-Acid Site Densities in Solid Acids", CATALYSIS LETTERS, Bd. 82, Nr. 3-4, Oktober 2002 (2002-10), Seiten 155-160, XP055508257, DOI: 10.1023/A:1020514911456
• LOVELESS B T ET AL: "Discrepancy between TPD- and FTIR-based measurements of Bronsted and Lewis acidity for sulfated zirconia", APPLIED CATALYSIS B: ENVIRONMENTAL, ELSEVIER, AMSTERDAM, NL, Bd. 84, Nr. 3-4, 11. Juni 2008 (2008-06-11), Seiten 591-597, XP025562320, ISSN: 0926-3373, DOI: 10.1016/J.APCATB.2008.05.025 [gefunden am 2008-06-11]
• PHUNG THANH KHOA ET AL: "On the use of infrared spectrometer as detector for Temperature Programmed (TP) techniques in catalysts characterization", JOURNAL OF INDUSTRIAL AND ENGINEERING CHEMISTRY, Bd. 47, 6. Dezember 2017 (2017-12-06), Seiten 288-296, XP029889918, ISSN: 1226-086X, DOI: 10.1016/J.JIEC.2016.11.045

EP 3 540 435 B1

**Beschreibung**

Die vorliegende Erfindung betrifft ein Verfahren zur Bestimmung der Konzentration an sauren Zentren eines Feststoffes, die beispielsweise einen Rückschluss auf die katalytische Aktivität des Feststoffes zulässt.

Die bei der heterogenen Katalyse für die dort verwendeten Katalysatoren eingesetzten Feststoffe entfalten ihre katalytische Aktivität durch Wechselwirkungen an der Oberfläche. Dabei können Oberflächenatome (bspw. Oberflächen-Aluminiumatome in Alumosilicaten) ein saures Zentrum (auch: Säurezentrum), beispielsweise ein Brönsted-Säurezentrum oder Lewis-Säurezentrum darstellen. Die Menge bzw. die Konzentration oder Dichte dieser sauren Zentren wird im Hinblick auf die katalytische Aktivität üblicherweise als der wesentliche Parameter angesehen.

Ein Brönsted-Säurezentrum ist ein Zentrum, welches ein Proton abgeben kann. Bei Alumosilicaten kann dies beispielsweise ein trivalentes Al-Atom in direkter Nachbarschaft zu einer Silanol-Gruppe, die dann das Proton abgibt, sein. Ein Lewis-Säurezentrum ist ein Elektronen-Akzeptor, im Falle von Alumosilicaten beispielsweise trivalente Oberflächen-Aluminiumatome, Silicat-Fehlstellen an der Oberfläche ($Si^+$ mit nur drei Bindungen) oder auch Rückstände aus der Synthese, wie $Na^+$ Kationen, also Zentren, die Elektronen aufnehmen können (z. B. das freie Elektronenpaar einer Aminverbindung).

Im Stand der Technik sind Verfahren zur Bestimmung der Konzentration an sauren Zentren bekannt. Ein Verfahren ist die temperaturprogrammierte Desorption von Ammoniak (Ammoniak-TPD oder TPAD). Dabei wird ein Adsorptiv, das sogenannte Sondenmolekül (in diesem Fall Ammoniak) unter definierten Bedingungen zunächst auf der Feststoffprobe adsorbiert. Bei erhöhter Temperatur wird dann oft Helium über die Feststoffprobe geleitet, um physisorbierte Ammoniakmoleküle auszutreiben. Anschließend wird die temperaturinduzierte Desorption dieser Sonde verfolgt, wobei die Temperatur langsam erhöht wird (mit etwa 1 oder mehreren K/min) und die dabei frei werdenden Ammoniakmoleküle detektiert, z. B. über einen Wärmeleitfähigkeitsdetektor. Jedes detektierte Ammoniakmolekül steht bei dieser Analyse für ein Säurezentrum. Je geringer die Desorptionstemperatur, desto schwächer das Säurezentrum.

Das Problem dieses Verfahrens ist jedoch, dass die Interpretation der Ergebnisse des Ammoniak-TPD-Verfahren bisweilen kompliziert ist. In der Literatur wurde deshalb ein TPD-Verfahren vorgeschlagen, bei dem als Sondenmolekül ein Amin wie n-Propylamin eingesetzt wird, um ausschließlich die Menge bzw. die Konzentration der Brönsted-Säurezentren zu bestimmen (vgl. Kresnawahjuesa, O., Gorte, R., de Oliveira, D. et al., "A Simple, Inexpensive, and Reliable Method for Measuring Brønsted-Acid Site Densities in Solid Acids", Catalysis Letters, Volume 82, Issue 3-4 (2002), S. 155-160). Bei diesem Verfahren wird der Abbau von Aminen durch Brönsted-Säuren (die sogenannte Hoffmann-Eliminierung) ausgenutzt, bei der aus dem Amin beispielsweise ein Alken und Ammoniak gebildet wird. Die resultierenden Alkene und Ammoniak werden dann mittels Massenspektrometrie oder Gaschromatographie detektiert, um daraus die Menge bzw. die Konzentration der Brönsted-Säurezentren zu ermitteln.

Ferner offenbart Loveless, B. et al.: "Discrepancy between TPD- and FTIR-based measurements of Bronsted and Lewis acidity for sulfated zirconia", Applied Catalysis B: Environmental, Bd. 84, Nr. 3-4, (2008-06-11), Seiten 591-597, die Nutzung FTIR-basierter Messungen direkt an mit Aminen gesättigten Katalysatoren, sowie einer TPD auch mit Pyridin und anderer Varianten.

Das Problem aller TPD-Verfahren bleibt jedoch die vergleichsweise lange Dauer des Analyseverfahrens, die auf den vergleichsweise kleinen Heizraten von wenigen K/min beruht.

Die zugrundeliegende Aufgabe der vorliegenden Erfindung war deshalb die Bereitstellung eines Bestimmungsverfahrens für Säurezentren von Feststoffen, welches einfach und schnell durchgeführt werden kann. Durch ein verkürztes Messverfahren können viele Proben in kurzer Zeit analysiert werden und beispielsweise auf ihre Eignung für bestimmte Einsatzzwecke kostengünstig untersucht werden.

Die erfindungsgemäße Aufgabe konnte dadurch gelöst werden, dass nicht die temperaturprogrammierte Desorption untersucht wird, sondern adsorbierte Amine bei hoher Temperatur zersetzt werden, wodurch sich CO und/oder $CO_2$ bilden, welche mit einem entsprechenden Messgerät als TOC (total organic carbon) quantifiziert werden können. Die Lösung der Aufgabe ergibt sich insbesondere durch das in Anspruch 1 beschriebene Verfahren. Bevorzugte Ausgestaltungen sind in den Unteransprüchen angeben.

Das erfindungsgemäße Verfahren zur Bestimmung der Konzentration an sauren Zentren eines Feststoffes umfasst mindestens die folgenden Schritte:

a) Beaufschlagen eines zu untersuchenden Feststoff, welcher eine Zersetzungstemperatur größer oder gleich 400 °C aufweist, mit einer Amin-Verbindung, wodurch die Amin-Verbindung mit den sauren Zentren des Feststoffes wechselwirkt und dadurch ein mit Amin beaufschlagter Feststoff, bei dem an die sauren Zentren eine Amin-Verbindung adsorbiert ist, entsteht;

b) Erhitzen des getrockneten mit Amin beaufschlagten Feststoffes auf eine Temperatur von mehr als 1000 °C, vorzugsweise mehr als 1150 °C, besonders bevorzugt mehr als 1300 °C, wodurch das mit dem Feststoff wechselwirkende Amin zu $CO/CO_2$ umgesetzt wird;

c) Quantitative Bestimmung des Kohlenstoffgehalts (TOC-Wert) mittels IR-Spektroskopie des entstandenen $CO/CO_2$;

d) Bestimmung der Masse der adsorbierten Amin-Verbindung anhand des Kohlenstoffgehalts;

e) Bestimmung der Konzentration an sauren Zentren aus der nach Schritt d) ermittelten Masse der Amin-Verbindung, unter der Annahme, dass ein Amin mit einem sauren Zentrum wechselwirkt.

Der zu untersuchende Feststoff, der mit dem erfindungsgemäßen Verfahren untersucht werden kann, sollte eine Zersetzungstemperatur von größer oder gleich 400 °C aufweisen, damit der Feststoff bei der nachfolgenden Trocknung sich nicht zersetzt. Der zu untersuchende Feststoff sollte außerdem vorzugsweise bei Temperaturen < 1000 °C, vorzugsweise < 1150 °C, besonders bevorzugt < 1300 °C keinen Kohlenstoff in Form von $CO/CO_2$ freisetzen und/oder sich zersetzen. In einer bevorzugten Ausführungsform wird der zu untersuchende Feststoff aus der Gruppe, bestehend aus Alumosilicaten, Aluminiumoxid, Siliziumdioxid, mit anorganischen Säuren behandeltem Siliziumdioxid und Siliziumcarbid, Mischungen davon, ausgewählt. Besonders bevorzugt sind Feststoffe, die in der heterogenen Katalyse als Katalysatoren oder als Supportmaterialien für Katalysatoren eingesetzt werden können, wie Alumosilicate.

Das Beaufschlagen des zu untersuchenden Feststoffes kann auf unterschiedliche Weise erfolgen, entweder durch Inkontaktbringen des zu untersuchenden Feststoffes mit einer bei Raumtemperatur gasförmigen Amin-Verbindung, beispielsweise in einem Druckbehälter, optional unter Verwendung eines Lösemittels in dem sich die gasförmige Amin-Verbindung löst, oder dadurch dass der zu untersuchende Feststoff mit einer bei Raumtemperatur flüssigen Amin-Verbindung in Kontakt gebracht wird. Das Inkontaktbringen des zu untersuchenden Feststoffes mit der Amin-Verbindung kann optional unter Verwendung eines Lösemittels erfolgen.

Die eingesetzte Amin-Verbindung kann insbesondere ein primäres Amin sein, wodurch sterische Hinderungen der Wechselwirkungen mit den sauren Zentren des zu untersuchenden Feststoffes minimiert werden können. Zudem weisen primäre Amine üblicherweise eine vergleichsweise hohe Basizität auf, wodurch diese auch an schwächer sauren Zentren adsorbieren können. Weiterhin bevorzugt ist die Amin-Verbindung Monoalkylamin, wobei die Alkylgruppe 1 bis 8 Kohlenstoffatome, vorzugsweise 1 bis 6 Kohlenstoffatome, besonders bevorzugt 1 bis 4 Kohlenstoffatome aufweist. In einer besonders bevorzugten Ausführungsform werden als Amin-Verbindung Methylamin, Ethylamin, n-Propylamin oder iso-Propylamin eingesetzt.

Wird der zu untersuchende Feststoff in Schritt a) mit einer bei Raumtemperatur flüssigen Amin-Verbindung beaufschlagt, sind vorzugsweise die folgenden Schritte a1) und a2) vor dem Schritt b) durchzuführen:

a1) Herstellen einer Dispersion aus dem zu untersuchenden Feststoff, welcher eine Zersetzungstemperatur größer oder gleich 400 °C aufweist, und einer bei Raumtemperatur flüssigen Amin-Verbindung und optional einem organischen Lösemittel, wodurch ein mit Amin beaufschlagter Feststoff, bei dem an die sauren Zentren eine Amin-Verbindung adsorbiert ist, entsteht;

a2) Abfiltrieren und Trocknen des mit dem Amin beaufschlagten Feststoffes bei 40 °C bis 400 °C, um das gegebenenfalls vorhandene Lösemittel und die überschüssige Amin-Verbindung, also die, die nicht mit den Säurezentren wechselwirkt, auszutragen

Die Herstellung der Dispersion in Schritt a1) kann mit üblichen Verfahren erfolgen, um einen Feststoff in einer Flüssigkeit zu dispergieren, beispielsweise durch Rühren mit einem Rührwerk oder mit einem geeigneten Rührstab oder Schütteln des Mischgefäßes. Das optional zu verwendende Lösemittel ist ein organisches Lösemittel, vorzugsweise ein unpolares organisches Lösemittel. Das optional eingesetzte Lösemittel weist vorzugsweise einen Siedepunkt von kleiner 200 °C auf, damit es im nachfolgenden Trocknungsschritt a2) bei relativ gemäßigten Temperaturbedingungen entfernt werden kann.

In einer bevorzugten Ausführungsform wird das optional vorhandene organische Lösemittel aus der Gruppe, bestehend aus Alkanen mit 5 bis 12 Kohlenstoffatomen, Cycloalkanen mit 5 bis 12 Kohlenstoffatomen, heteroatomfreien aromatischen Kohlenwasserstoffen mit 6 bis 12 Kohlenstoffatomen und Mischungen von zwei oder mehr davon, ausgewählt. Besonders bevorzugt wird n-Hexan als Lösemittel verwendet.

Nach Herstellung der Dispersion in Schritt a1) kann die resultierende Dispersion mit zugegebener Amin-Verbindung über einen Zeitraum von 1 bis 72 Stunden, vorzugsweise 2 bis 18 Stunden stehen gelassen werden. Das kann notwendig sein, damit sichergestellt ist, dass an alle sauren Zentren eine Amin-Verbindung adsorbiert ist.

Bevor der mit dem Amin beaufschlagte Feststoff in Schritt a2) getrocknet wird, kann der Feststoff zusätzlich mit einem organischen Lösemittel gewaschen werden, um gegebenenfalls vorhandenes überschüssiges Amin, welches zu Ungenauigkeiten bei der nachfolgenden Bestimmung der sauren Zentren des Feststoffes führen könnte, auszutragen. Das organische Lösemittel kann mit dem zur Herstellung der Dispersion in Schritt a1) optional verwendeten organischen Lösemittel identisch sein, kann aber auch zu dem zur Herstellung der Dispersion verwendeten organischen Lösemittel unterschiedlich sein.

Das organische Lösemittel, welches zum Waschen des mit Amin beaufschlagten Feststoffes verwendet werden kann, ist vorzugsweise ein unpolares organisches Lösemittel. Weiterhin bevorzugt kann das organische Lösemittel aus der Gruppe, bestehend aus Alkanen mit 5 bis 12 Kohlenstoffatomen, Cycloalkanen mit 5 bis 12 Kohlenstoffatomen, heteroatomfreien aromatischen Kohlenwasserstoffen mit 6 bis 12 Kohlenstoffatomen und Mischungen von zwei oder mehr davon, ausgewählt. Besonders bevorzugt kann n-Hexan zum Waschen verwendet werden.

Im nachfolgenden Schritt a2), das heißt nach dem Beaufschlagen des zu untersuchenden Feststoffes mit der Amin-Verbindung oder nach dem optionalen Waschen des mit dem Amin beaufschlagten Feststoffes, wird der mit dem Amin beaufschlagte Feststoff zunächst nach üblichen Verfahren abfiltriert und dann bei einer Temperatur von 40 °C bis 400 °C, vorzugsweise 80 °C bis 375 °C getrocknet. Die Trocknung erfolgt vorzugsweise über einen Zeitraum von 1 bis 24 Stunden, besonders bevorzugt 2 bis 18 Stunden.

Nachdem der mit dem Amin beaufschlagte Feststoff getrocknet worden ist, wird der Feststoff in Schritt b) auf eine Temperatur von mehr als 1000 °C, vorzugsweise mehr als 1150 °C, besonders bevorzugt mehr als 1300 °C erhitzt. Die Amin-Verbindung, mit dem der Feststoff beaufschlagt wurde und die mit den sauren Zentren des Feststoffes wechselwirkt bzw. an den sauren Zentren des Feststoffes adsorbiert ist, wird dadurch zersetzt, wobei CO und/oder $CO_2$, jedoch kein Ruß o.ä. entstehen.

Zum Erhitzen in Schritt b) kann eine definierte Menge (z. B. 0,1g bis 2g) des mit dem Amin beaufschlagten Feststoffes in ein handelsübliches Gefäß gefüllt werden, welches der Temperatur, auf die der mit dem Amin beaufschlagte Feststoff erhitzt wird, standhält, beispielsweise einen Keramik-Tiegel. Während des Erhitzens in Schritt b) wird vorzugsweise Luft über die Probe geleitet, besonders bevorzugt aus allen Richtungen, um zu erreichen, dass durch den dadurch zusätzlich zugeführten (Luft-)Sauerstoff bei der Zersetzung der Amin-Verbindung vorrangig $CO_2$ gebildet wird.

Das beim Erhitzen in Schritt b) gebildete CO bzw. $CO_2$ wird in Schritt c) quantitativ mittels IR-Spektroskopie analysiert. Bei der quantitativen Analyse, die mit einem handelsüblichen Analysegerät, beispielsweise einem Kohlenstoff/Schwefel-Analysator durchgeführt werden kann, wird nicht zwangsläufig die Menge des gebildeten $CO/CO_2$ für den Anwender ausgegeben, sondern gegebenenfalls auch oder nur der TOC-Wert (total organic carbon), also der absolute Anteil an organischem Kohlenstoff der analysierten mit dem Amin beaufschlagten Feststoffprobe. Der TOC-Wert wird vom Analysegerät üblicherweise in der Einheit Masse Kohlenstoff pro Gramm der Probe ausgegeben. Die Analysedauer für eine Feststoffprobe beträgt im Gegensatz zu bekannten Verfahren vorzugsweise weniger als 5 Minuten.

Mit diesem TOC-Wert kann zunächst die Masse der auf dem Feststoff beaufschlagten und dort an den sauren Zentren adsorbierten Amin-Verbindung ($m_{Amin}$) berechnet werden. Hierfür wird die Stoffmenge an Kohlenstoff berechnet, wobei die in Schritt c) bestimmte Masse an Kohlenstoff (TOC-Wert: $m_{TOC}$) durch die molare Masse von Kohlenstoff ($M_C$) geteilt wird. Die erhaltene Stoffmenge an Kohlenstoff gibt die Anzahl der C-Atome auf der Probe an. Um die Anzahl der C-Atome auf die Stoffmenge der Amin-Verbindung übertragen zu können, wird die berechnete Stoffmenge von Kohlenstoff durch die Anzahl der Kohlenstoffatome (Stöchiometriekoeffizent $v_C$) in der verwendeten Amin-Verbindung geteilt. Wird beispielsweise n-Propylamin als Amin-Verbindung verwendet, beträgt der Stöchiometriekoeffizient ($v_C$) 3, da aus jedem der drei Kohlenstoffatome des n-Propylamin beim Erhitzen ein $CO_2$- bzw. CO-Molekül gebildet wird. Mithilfe der molaren Masse der verwendeten Amin-Verdingung ($M_{Amin}$) kann dann die Masse der auf dem Feststoff beaufschlagten und dort an den sauren Zentren adsorbierten Amin-Verbindung mit folgender Formel (1) errechnet werden:

$$m_{Amin} = \frac{1}{v_C} \times \frac{m_{TOC}}{M_C} \times M_{Amin} \qquad (1)$$

Für eine exakte Berechnung der Konzentration an sauren Zentren, hier sowohl die Brönsted-Säurezentrum und die Lewis-Säurezentren, muss auch die genaue Masse des untersuchten Feststoffes ($m_{Feststoff}$) berechnet werden, da die bei der Analyse in Schritt c) untersuchte definierte Menge der mit Amin-beaufschlagten Feststoffprobe ($m_{probe}$) sich aus der Masse des Feststoffes ($m_{Feststoff}$) und der Masse der adsorbierten Amin-Verbindung ($m_{Amin}$) zusammensetzt. Die aus der Formel (1) errechnete Masse der adsorbierten Amin-Verbindung wird folglich von der bei der Analyse in Schritt c) eingesetzten definierten Menge der Feststoffprobe (inklusive der Masse der Amin-Verbindung) gemäß Formel (2) abgezogen, um die genaue Masse des untersuchten Feststoffes zu ermitteln:

$$m_{Feststoff} = m_{Probe} - m_{Amin} \qquad (2)$$

Für die Berechnung der Konzentration an sauren Zentren ($c_{Säurezentren}$) muss zunächst davon ausgegangen werden, dass pro saurem Zentrum genau ein Molekül der Amin-Verbindung adsorbiert ist. Da die Probe vor der Messung bei mindestens 40 °C, vorzugsweise mindestens 80 °C getrocknet und vorher optional mit einem Lösemittel gewaschen wurde, ist eine solche Annahme durchaus zutreffend, da physisorbierte Moleküle der Amin-Verbindung verdrängt werden. Die Konzentration an sauren Zentren ergibt sich dann aus der berechneten Masse der adsorbierten Amin-Verbindung ($m_{Amin}$), die durch die molare Masse der Amin-Verbindung ($M_{Amin}$) und die genaue Masse des untersuchten Feststoffes ($m_{Feststoff}$) geteilt wird. Da die Konzentration der sauren Zentren üblicherweise in mmol/g angegeben wird, muss das Ergebnis vorher noch mit 1000 multipliziert werden. Daraus ergibt sich für die Berechnung der Konzentration an sauren Zentren die nachfolgende Formel (3):

$$c_{Säurezentren}\left(\frac{mmol}{g}\right) = \frac{m_{Amin}}{M_{Amin} \times m_{Feststoff}} \times 1000 \qquad (3)$$

Die Konzentration der Säurezentren kann natürlich auch in einer beliebigen anderen Einheit angegeben werden. Soll das Ergebnis in einer anderen Einheit angegeben werden, müssen gegebenenfalls andere Umrechnungsfaktoren eingesetzt werden.

Beispiel:

Bestimmung der Konzentration an sauren Zentren bei einem Alumosilicat anhand des erfindungsgemäßen Verfahrens

Das Alumosilicat (Si/Al-Verhältnis von 6,4, BET-Oberfläche ca. 300 m²/g) wurde zunächst bei 550 °C calciniert, damit Wasser und eventuell Ammoniak ausgetrieben werden. Danach wurde das Alumosilicat in eine Probenflasche gegeben und mit n-Hexan aufgefüllt und geschüttelt, um das Alumosilicat zu dispergieren. Zu der Dispersion wurde anschließend n-Propylamin gegeben und stehen gelassen, wobei das n-Propylamin mit dem Aluminium an der Oberfläche des Alumosilicats wechselwirkt und an den sauren Zentren adsorbiert wird. Nach der Adsorption des n-Propylamins wurde das Alumosilicat mit Hexan gewaschen, um gegebenenfalls vorhandenes überschüssiges Amin auszutragen, und danach das mit Amin beaufschlagte Alumosilicat abfiltriert. Das mit Amin beaufschlagte Alumosilicat wurde anschließend bei 350 °C getrocknet.

Für die Bestimmung des TOC-Wertes wird die mit Amin beaufschlagte Feststoffprobe in einen Keramik-Tiegel gegeben und auf 1300 °C erhitzt. Sämtliche organische Verbindungen (in diesem Fall das n-Propylamin) reagieren dadurch zu CO und/oder $CO_2$, was dann IR-spektroskopisch quantitativ analysiert wurde. Die Messung wurde mit dem "Kohlenstoff und Schwefel Analysator CS-580A" der Firma ELTRA durchgeführt (Messzeit pro Probe kleiner 5 min). Anhand dem ermittelten Kohlenstoffgehalt lässt sich die Konzentration an sauren Zentren nach den in der Beschreibung genannten Formeln (1) bis (3) errechnen. Für die Konzentration an sauren Zentren wurden 0,31 mmol/g errechnet.

Bestimmung der Konzentration an sauren Zentren bei einem Alumosilicat anhand des Ammoniak-TPD-Verfahrens

Hierfür wurden etwa 1 g Alumosilicat als Probensubstanz in eine Messzelle gegeben. In dieser Messzelle wurde die Probe calciniert, d. h. auf etwa 550 °C geheizt, wodurch Wasser und andere Komponenten (wie z. B. Ammoniak) aus der Probe ausgetrieben werden. Während dieser Calcination wurde die Probe mit Argon durchströmt, damit die frei werdenden Komponenten besser abtransportiert werden können. Anschließend wurde die Probe auf Raumtemperatur abgekühlt. Anschließend wurde Ammoniak für etwa 1 Minute über die Probe geleitet, um die Säurezentren mit Ammoniak zu belegen. Danach wurde Helium für etwa 30 Minuten bei 100 °C über die Probe geleitet, um physisorbierte Ammoniak-Moleküle zu entfernen. Anschließend wurde die Temperatur langsam erhöht (etwa 1 K/min) und der dabei freiwerdende Ammoniak detektiert (mit dem TPDRO 1100 von Thermo Scientific). Für die Berechnung der Konzentration der sauren Zentren wurde ebenfalls angenommen, dass je ein Ammoniak-Molekül an ein saures Zentrum adsorbiert ist. Daraus konnte eine Konzentration an sauren Zentren von 0,31 mmol/g ermittelt werden.

Es zeigt sich also, dass das erfindungsgemäße Verfahren als Alternative zu den bekannten TPD-Verfahren geeignet ist. Als Vorteil ergeben sich eine deutlich geringere Messdauer von nur wenigen Minuten bei dem erfindungsgemäßen Verfahren und eine deutlich einfachere Auswertung der Versuchsergebnisse zur Berechnung der Konzentration an sauren Zentren.

**Patentansprüche**

1.   Verfahren zur Bestimmung der Konzentration an sauren Zentren eines Feststoffes, wobei das Verfahren folgende Schritte umfasst:

a) Beaufschlagen eines zu untersuchenden Feststoff, welcher eine Zersetzungstemperatur größer oder gleich 400 °C aufweist; mit einer Amin-Verbindung, wodurch die Amin-Verbindung mit den sauren Zentren des Feststoffes wechselwirkt und dadurch ein mit Amin beaufschlagter Feststoff, bei dem an die sauren Zentren eine Amin-Verbindung adsorbiert ist, entsteht;

b) Erhitzen des getrockneten mit Amin beaufschlagten Feststoffes auf eine Temperatur von mehr als 1000 °C, vorzugsweise mehr als 1100 °C, besonders bevorzugt mehr als 1200 °C, wodurch das auf dem Feststoff beaufschlagte Amin zu $CO/CO_2$ reagiert wird;

c) Quantitative Bestimmung des Kohlenstoffgehalts (TOC-Wert) mittels IR-Spektroskopie des entstandenen $CO/CO_2$;

d) Bestimmung der Masse der adsorbierten Amin-Verbindung anhand des Kohlenstoffgehalts;

e) Bestimmung der Konzentration an sauren Zentren in mmol/g aus der nach Schritt d) ermittelten Masse der Amin-Verbindung, unter der Annahme, dass ein Amin mit einem sauren Zentrum wechselwirkt.

2. Verfahren nach Anspruch 1, wobei der zu untersuchende Feststoff aus der Gruppe, bestehend aus Alumosilicaten, Aluminiumoxid, Siliziumdioxid, mit anorganischen Säuren behandeltem Siliziumdioxid, Siliziumcarbid und Mischungen davon, ausgewählt wird.

3. Verfahren nach Anspruch 1 oder 2, wobei die Amin-Verbindung bei Raumtemperatur flüssig oder gasförmig ist.

4. Verfahren nach Anspruch 3, wobei die Amin-Verbindung ein primäres Amin ist.

5. Verfahren nach Anspruch 4, wobei die Amin-Verbindung ein Alkylamin, vorzugsweise ein Monoalkylamin ist, wobei die Alkylgruppe 1 bis 8 Kohlenstoffatome, vorzugsweise 1 bis 6 Kohlenstoffatomen, besonders bevorzugt 1 bis 4 Kohlenstoffatome aufweist.

6. Verfahren nach Anspruch 5, wobei die Amin-Verbindung Methylamin, Ethylamin, n-Propylamin oder iso-Propylamin ist.

7. Verfahren nach einem der Ansprüche 1 bis 6, wobei, wenn der zu untersuchende Feststoff mit einer bei Raumtemperatur flüssigen Amin-Verbindung beaufschlagt wird, die folgenden Schritte a1) und a2) vor dem Schritt b) durchzuführen sind:

a1) Herstellen einer Dispersion aus dem zu untersuchenden Feststoff, welcher eine Zersetzungstemperatur größer oder gleich 400 °C aufweist, und einer bei Raumtemperatur flüssigen Amin-Verbindung und optional einem organischen Lösemittel, wodurch ein mit Amin beaufschlagter Feststoff, bei dem an die sauren Zentren eine Amin-Verbindung adsorbiert ist, entsteht; und

a2) Abfiltrieren und Trocknen des mit dem Amin beaufschlagten Feststoffes bei 40 °C bis 400 °C, um das gegebenenfalls vorhandene Lösemittel und die überschüssige Amin-Verbindung auszutragen.

8. Verfahren nach Anspruch 7, wobei der mit Amin beaufschlagte Feststoff zwischen Schritt a2) und Schritt b) mit einem organischen Lösemittel gewaschen wird, um überschüssiges Amin auszutragen.

9. Verfahren nach einem der Ansprüche 7 oder 8, wobei das organische Lösemittel ein unpolares organisches Lösemittel ist.

10. Verfahren nach Anspruch 9, wobei das organische Lösemittel aus der Gruppe, bestehend aus einem Alkan mit 5 bis 12 Kohlenstoffatomen, einem Cycloalkan mit 5 bis 12 Kohlenstoffatomen, einem heteroatomfreien aromatischen Kohlenwasserstoff mit 6 bis 12 Kohlenstoffatomen und Mischungen von zwei oder davon, ausgewählt wird.

11. Verfahren nach einem der Ansprüche 7, wobei die Trocknung in Schritt a2) über einen Zeitraum von 1 bis 24 Stunden, vorzugsweise 2 bis 18 Stunden erfolgt.

12. Verfahren nach einem der Ansprüche 1 bis 11, wobei die Bestimmung des Kohlenstoffgehalts in Schritt c) mit einem Kohlenstoff/Schwefel Analysator durchgeführt wird.

**Claims**

1. Method for determining the concentration of acidic sites of a solid, wherein the method comprises the following steps:

a) treating a solid to be investigated, which has a decomposition temperature of greater than or equal to 400°C, with an amine compound, by means of which the amine compound interacts with the acidic sites of the solid and as a result an amine-treated solid arises, in which an amine compound is adsorbed at the acidic sites,

b) heating the dried amine-treated solid to a temperature of greater than 1000°C, preferably greater than 1100°C, particularly preferably greater than 1200°C, as a result of which the amine applied to the solid is reacted to give $CO/CO_2$;

c) quantitative determination of the carbon content (TOC value) using IR spectroscopy of the resulting $CO/CO_2$;

d) determining the mass of the adsorbed amine compound using the carbon content;

e) determining the concentration of acidic sites in mmol/g from the mass of the amine compound determined according to step d), with the assumption that one amine interacts with one acidic site.

2. Method according to Claim 1, wherein the solid to be investigated is selected from the group consisting of aluminosilicates, aluminium oxide, silicon dioxide, silicon dioxide treated with inorganic acids, silicon carbide and mixtures thereof.

3. Method according to Claim 1 or 2, wherein the amine compound is liquid or gaseous at room temperature.

4. Method according to Claim 3, wherein the amine compound is a primary amine.

5. Method according to Claim 4, wherein the amine compound is an alkylamine, preferably a monoalkylamine, wherein the alkyl group has 1 to 8 carbon atoms, preferably 1 to 6 carbon atoms, particularly preferably 1 to 4 carbon atoms.

6. Method according to Claim 5, wherein the amine compound is methylamine, ethylamine, n-propylamine or isopropylamine.

7. Method according to one of Claims 1 to 6, wherein, if the solid to be investigated is treated with an amine compound that is liquid at room temperature, the following steps a1) and a2) are to be carried out before step b):

a1) preparing a dispersion of the solid to be investigated, which has a decomposition temperature of greater than or equal to 400°C, and an amine compound that is liquid at room temperature, and optionally an organic solvent, by means of which an amine-treated solid arises, in which an amine compound is adsorbed at the acidic sites; and

a2) filtering off and drying the amine-treated solid at 40°C to 400°C, in order to discharge the optionally present solvent and the excess amine compound.

8. Method according to Claim 7, wherein the amine-treated solid is washed between step a2) and step b) with an organic solvent, in order to discharge excess amine.

9. Method according to one of Claims 7 and 8, wherein the organic solvent is a nonpolar organic solvent.

10. Method according to Claim 9, wherein the organic solvent is selected from the group consisting of an alkane having 5 to 12 carbon atoms, a cycloalkane having 5 to 12 carbon atoms, a heteroatom-free aromatic hydrocarbon having 6 to 12 carbon atoms and mixtures of two or thereof.

11. Method according to one of Claims 7, wherein the drying in step a2) is carried out over a period of time of 1 to 24 hours, preferably 2 to 18 hours.

12. Method according to one of Claims 1 to 11, wherein the carbon content is determined in step c) using a carbon/sulfur analyzer.

## Revendications

1. Procédé pour la détermination de la concentration en centres acides d'un solide, le procédé comprenant les étapes suivantes :

a) alimentation d'un solide à étudier, qui présente une température de décomposition supérieure ou égale à 400 °C ; avec un composé d'amine, le composé d'amine interagissant avec les centres acides du solide et ainsi, un solide alimenté par une amine est obtenu, dans lequel un composé d'amine est adsorbé au niveau des centres acides ;

b) chauffage du solide séché alimenté par une amine, à une température de plus de 1 000 °C, de préférence plus de 1 100 °C, particulièrement préférablement plus de 1 200 °C, l'amine alimentée sur le solide réagissant pour donner du $CO/CO_2$ ;

c) détermination quantitative de la teneur en carbone (valeur TOC) au moyen de spectroscopie IR du $CO/CO_2$ formé ;

d) détermination de la masse du composé d'amine adsorbé à partir de la teneur en carbone ;

e) détermination de la concentration en centres acides en mmole/g à partir de la masse du composé d'amine déterminée après l'étape d), en supposant qu'une amine interagit avec un centre acide.

2. Procédé selon la revendication 1, le solide à étudier étant choisi dans le groupe constitué par les aluminosilicates, l'oxyde d'aluminium, le dioxyde de silicium, le dioxyde de silicium traité par des acides inorganiques, le carbure de silicium et des mélanges correspondants.

3. Procédé selon la revendication 1 ou 2, le composé d'amine étant liquide ou gazeux à température ambiante.

4. Procédé selon la revendication 3, le composé d'amine étant une amine primaire.

5. Procédé selon la revendication 4, le composé d'amine étant une alkylamine, de préférence une monoalkylamine, le groupe alkyle présentant 1 à 8 atome(s) de carbone, de préférence 1 à 6 atome(s) de carbone, particulièrement préférablement 1 à 4 atome(s) de carbone.

6. Procédé selon la revendication 5, le composé d'amine étant la méthylamine, l'éthylamine, la n-propylamine ou l'iso-propylamine.

7. Procédé selon l'une quelconque des revendications 1 à 6, où lorsque le solide à étudier est alimenté avec un composé d'amine liquide à température ambiante, les étapes suivantes a1) et a2) sont à réaliser avant l'étape b) :

a1) préparation d'une dispersion à partir du solide à étudier, qui présente une température de décomposition supérieure ou égale à 400 °C, et d'un composé d'amine liquide à température ambiante et éventuellement d'un solvant organique, un solide alimenté par une amine étant ainsi obtenu, dans lequel un composé d'amine est adsorbé au niveau des centres acides ; et

a2) filtration et séchage du solide alimenté par l'aminé à une température de 40 °C à 400 °C, afin d'évacuer le solvant éventuellement présent et le composé d'amine en excès.

8. Procédé selon la revendication 7, le solide alimenté par une amine étant lavé entre l'étape a2) et l'étape b) avec un solvant organique, afin d'évacuer l'amine en excès.

9. Procédé selon l'une quelconque des revendications 7 et 8, le solvant organique étant un solvant organique apolaire.

10. Procédé selon la revendication 9, le solvant organique étant choisi dans le groupe constitué par un alcane comportant 5 à 12 atomes de carbone, un cycloalcane comportant 5 à 12 atomes de carbone, un hydrocarbure aromatique exempt d'hétéroatome comportant 6 à 12 atomes de carbone et des mélanges de deux ou de ceux-ci.

11. Procédé selon l'une des revendications 7, le séchage dans l'étape a2) étant réalisé sur une période de temps de 1 à 24 heures, de préférence 2 à 18 heures.

12. Procédé selon l'une quelconque des revendications 1 à 11, la détermination de la teneur en carbone dans l'étape c) étant réalisée avec un analyseur carbone/soufre.

**IN DER BESCHREIBUNG AUFGEFÜHRTE DOKUMENTE**

**In der Beschreibung aufgeführte Nicht-Patentliteratur**

- **KRESNAWAHJUESA, O. ; GORTE, R. ; DE OL-IVEIRA, D. et al.** A Simple, Inexpensive, and Reliable Method for Measuring Brønsted-Acid Site Densities in Solid Acids. *Catalysis Letters,* 2002, vol. 82 (3-4), 155-160

- **LOVELESS, B. et al.** Discrepancy between TPD- and FTIR-based measurements of Bronsted and Lewis acidity for sulfated zirconia. *Applied Catalysis B: Environmental,* 11. Juni 2008, vol. 84 (3-4), 591-597